(19) Europäisches Patentamt
European Patent Office
Office européen des brevets

(11) Publication number: **0 396 251**
**A2**

(12) # EUROPEAN PATENT APPLICATION

(21) Application number: **90303361.1**

(22) Date of filing: **29.03.90**

(51) Int. Cl.$^5$: **A61K 31/20, A61K 31/23, A61K 31/16, A61K 31/495, A61K 31/05, A61K 31/35**

The title of the invention has been amended (Guidelines for Examination in the EPO, A-III, 7.3).

(30) Priority: **31.03.89 GB 8907308**
**25.08.89 JP 220144/89**

(43) Date of publication of application:
**07.11.90 Bulletin 90/45**

(84) Designated Contracting States:
**CH DE FR GB LI NL**

(71) Applicant: **NATIONAL UNIVERSITY OF SINGAPORE**
**10 Kent Ridge Crescent**
**Singapore 0511(SG)**

(72) Inventor: **Tan, Yin Hwee**
**10 Kent Ridge Crescent**
**Singapore 0511(SG)**
Inventor: **Lim, Louis**
**1 Wakefield Street**
**London WC1N 1PJ(GB)**

(74) Representative: **Woods, Geoffrey Corlett et al**
**J.A. KEMP & CO. 14 South Square Gray's Inn**
**London WC1R 5EU(GB)**

(54) **Use of fatty acids for the treatment of diseases associated with cytokines.**

(57) Use, in the preparation of a medicament for use in the treatment of a disease state associated with the endogenous presence and/or production of a cytokine, of arachidonic acid or an analogue thereof capable of interfering with the binding of the cytokine to the cellular receptor for the cytokine. The arachidonic acid or analogue thereof can be used to alleviate the symptoms of the common cold or of influenza.

EP 0 396 251 A2

Xerox Copy Centre

# METHOD OF TREATING A DISEASE STATE ASSOCIATED WITH THE ENDOGENOUS PRESENCE AND/OR PRODUCTION OF A CYTOKINE

The present invention relates to the treatment of a disease state associated with the endogenous presence and/or production of a cytokine, especially the treatment of the common cold and flu-like symptoms.

Little is known of any compound that can suppress the action of interferon except for actinomycin D, a known inhibitor of transcription which inhibits the transcription of interferon-induced genes, and for general inhibitors of protein synthesis which inhibit the translation of proteins necessary for the expression of the interferon-induced effects. These inhibitors of RNA and protein synthesis are generally non-specific and toxic to the host, prompting the search for substances that will inhibit the action of interferons.

Pottathil et al. reported that inhibitors of fatty acid cyclooxygenase suppress the establishment of the interferon-induced antiviral state. They proposed that inhibitors of fatty acid cyclooxygenase such as indomethacin and oxyphenylbutazone at 10μM inhibit interferon action because the fatty acid cyclooxygenase pathway is involved in mediating the action of interferon action on cells. However, this hypothesis was disputed by several research groups. It was not possible to confirm the observation that these inhibitors suppressed the interferon-induced antiviral state (Sarkar and Gupta; Takaoki et al. and see Table 1 below).

Sarkar and Gupta showed that interferon activity could only be suppressed by indomethacin or ibuprofen at high concentrations exceeding 1000μM. However, at these concentrations both drugs also began to inhibit total protein and RNA synthesis in the cells. We also could not measure any inhibitory effect of indomethacin (300μM) or oxyphenylbutazone (50μM) on the interferon-induced antiviral state in human foreskin fibroblasts in mouse L and in A9 cells (Table 1). Thus the proposal concerning a cyclooxygenase involvement in interferon action cannot be substantiated at the present time.

Even so, the role of arachidonate metabolism in mediating the action of interferons has been addressed by several groups but there is no clear evidence to support its involvement. Changes in arachidonate metabolism in cells treated with interferons (Fuse et al., Yaron et al., Eldor et al., Nielsen et al.) infer their role in the inflammatory effects of lymphokines. However, there are reports contradicting these observations as well (Pottathil et al., Dore-Duffy et al., Sarkar and Gupta, Takaoki et al.).

Similarly Elliot et al. reported that glucocorticoid hormones acted synergistically with human alpha interferon to enhance the sensitivity of Daudi cells to interferon. However, the involvement of phospholipase A2 in this interferon action is also not substantiable.

We have now discovered several compounds which can suppress the antiviral activity of human type I interferon ($\alpha$ and $\beta$ interferon) as well as the antiviral activity of a well known lymphokine, gamma interferon or type II interferon. These compounds and their derivatives can therefore be used to treat a disease state associated with the endogenous presence and/or production of a lymphokine.

Accordingly the present invention provides the use of arachidonic acid or an analogue thereof capable of interfering with the binding of a cytokine to the cellular receptor for the cytokine in the manufacture of a medicament for use in the treatment of a disease state associated with the endogenous presence and/or production of the cytokine.

It is therefore possible to use arachidonic acid and its analogues to counter the undesirable side effects of a host's endogenous cytokines on the host. There are several discussions on these effect (Bloom; Duffy; McPherson and Tan; Hayden et al (a); Scott et al and Abdi et al). We have discovered that, at least for some of the present compounds, the mechanism of their action in suppressing the antiviral activity of a cytokine, for example a lymphokine and specifically interferon, is mediated at the level of the interferon receptors, altering the binding of interferon to cells.

Interferon is one example of a cytokine whose effects on the host is closely associated with the disease state of the common cold (Scott et al.; Hayden et al (a)). This is one reason why interferon has not been used clinically as a drug to treat the common cold and the flu even though it is an antiviral (Douglas et al.; Hayden et al. (b)). Since however arachidonic acid and its analogues may effectively suppress the cellular action of interferon, any of them by themselves or in combination can be used to alleviate the undesirable symptoms and discomfort of patients suffering from the common cold or from influenza (flu) after the patient's endogenous interferon has produced its antiviral effect at the infected sites. Several groups have already shown that the transmission of the interferon signal into the cell is a very rapid event occurring within the first minute or few minutes following interferon-receptor interaction (Yap et al. (a) (b); McCoy & Strynadka; Faltynek et al.; Pfeffer and Reich; Tan et al.).

An analogue of arachidonic acid in the context of the present invention is a compound which is capable of interferring with the binding of a cytokine to the cellular receptor for the cytokine. The cytokine may be a

lymphokine. The cytokine may be type I or II interferon. Preferably an analogue should be capable of producing a percentage inhibition of the interferon-induced antiviral state of at least 50%, more preferably of at least 80% or at least 90%, according to the experimental protocol described below in Example 1.

One class of compounds which may be used in the present invention consists of monocarboxylic polyunsaturated fatty acids, their pharmaceutically acceptable salts, their $C_1$-$C_4$ alkyl esters, their amides and the mono-($C_1$-$C_4$ alkyl) amides. Typically the fatty acids have from 14 to 24 carbon atoms, for example from 18 to 22 carbon atoms and most preferably 20 carbon atoms. The carboxy group is a terminal carboxy group.

The acids are typically straight chain acids. The salt may be the sodium salt. The acids may contain up to 4, for example 3 or 4, conjugated or non-conjugated unsaturated carbon-carbon bonds. These bonds may be double or treble bonds or an acid may contain a combination of both. Suitable compounds include 5,8,11,14-eicosatetraenoic acid (arachidonic acid) 5,8,11,14-eicosatetraynoic acid, 5,8,11-eicosatriynoic acid, linoleic acid and their derivatives.

Other compounds which may be employed in the present invention are cis-1-acetyl-4-{4-[2-(2,4-dichlorophenyl)-2-imidazol-1-ylmethyl-1,3-dioxolan-4-ylmethoxy]phenyl}-piperazine (Ketoconazole); 4,4'-(2,3-dimethyltetramethylene)bis(benzene-1,2-diol) (Nordihydroguaiaretic acid);

**and Quercetin:** 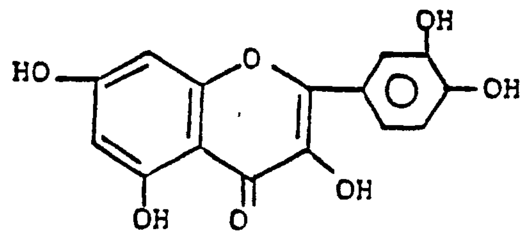 ;

and, where appropriate, their pharmaceutically acceptable salts

Typically, the analogues are also inhibitors of one or more of the cellular lipoxygenases and cyclooxygenase. They generally are compounds which interfere with the binding of ligands such as cytokines like types I and II interferon to their cellular receptors, for example by causing changes in the cell membrane. They may be hydrophobic and lipophilic in nature, and capable of dissolving in organic solvents such as dimethyl sulphoxide (DMSO) and ethyl alcohol. Water-soluble derivatives may be prepared, however, by forming salts.

Arachidonic acid and its analogues are used in the present invention as an inhibitor of an antiviral agent to treat viral diseases, for example the common cold and flu-like symptoms. Arachidonic acid is a precursor in the biochemical pathway(s) leading to production of intermediates thought to be associated with the inflammatory process of the host. However, its mode of action as a therapeutic agent as postulated here involves its ability to limit the action of interferon on cells after the establishment of an antiviral state in the affected cells. The initiation of the antiviral state is accomplished within a few minutes of cellular exposure to interferon. Arachidonic acid and its analogues are then employed according to the invention as attenuators of those aspects of interferon activity which are responsible for evoking flu-like symptoms.

Generally it is acknowledged but has not been verified that, in a viral infection, interferon constitutes the first line of defence of the host while the antibody response is the second line. For the normal population, the common cold and flu are not life-threatening diseases. Thus an appropriate strategy to deal with the common cold for such a population at large is to treat the flu-like symptoms of the disease after the endogenous interferon has accomplished its antiviral mission at the site of infection. This concept can be applied to other disease states where arachidonates and the other compounds can be used to counter the undesirable effects of other cytokines, bringing relief to a patient even though these compounds by themselves may not be efficacious against the disease.

Accordingly, the invention also provides a method of treating a patient with a disease state associated with the endogenous presence and/or production of a cytokine such as a lymphokine, which method comprises administering to the patient an effective amount of arachidonic acid or an analogue thereof.

Arachidonic acid or an analogue can therefore be used to alleviate a disease state. Symptoms may be treated. A disease state in a host, in particular a human subject, can be combated. Arachidonic acid or one of its analogues can be used to inhibit undesirable effects produced in a host by cytokines, for example body aches, malaise, pyrexia and flu-like symptoms. Each compound at appropriate doses and regimens can be used singly, in combination with a cytokine or with one or more other antiviral agents.

Arachidonic acid or one of its analogues may be presented for use in the present invention in the form

of a pharmaceutical composition also comprising a pharmaceutically acceptable carrier or diluent. Conventional carriers and diluents familiar to those skilled in the art may be employed. The composition may be in the form of nasal drops, a nasal spray, a balm, a cream, or tablets.

The arachidonic acid or analogue thereof may be applied topically as a nasal spray or nasal drops or in the form of a balm or cream. Alternatively, an oral route of administration may be selected. The dosage of arachidonic acid or analogues thereof given to a patient will depend on a variety of factors such as the disease state under treatment and the condition of the patient. For nasal delivery, however, arachidonic acid or an analogue may be administered at a concentration of 1 to 1000mM per 0.5 to 5 ml dose. From 0.5mg to 150mg of arachidonic acid or an analogue may be administered per dose orally. Doses may be given from 1 to 4 times per day as appropriate.

The following Examples illustrate the invention. Two Reference Examples are also provided. In the accompanying drawings:

Figures 1 and 2 show the results of the investigations in Example 2 into the kinetics of the inhibition of the interferon-induced antiviral state by arachidonic acid in human fibroblasts;

Figures 3 to 6 are Scatchard plots of human interferon binding data in human fibroblasts in Example 3; and

Figure 7 shows the results of the investigations in Example 4 into the effect of arachidonic acid and 5,8,11,14-eicosatetraynoic acid on interferon-induced transcription of 6/16 gene.

Reference Example 1: The effect of indomethacin and oxyphenylbutazone on the induction of antiviral state by human α interferon in human fibroblasts and by mouse interferon in mouse L or A9 cells

Human fibroblasts, mouse L or A9 cells were grown to confluency in the wells of 96 well Micro-test II plates. The confluent monolayer of cells in each micro-well was incubated with 200μl of growth medium containing 10% fetal calf serum and the indicated concentrations of the compound or without the compound in the control experiments for 6 hours.at 37° C. Thereafter the human or mouse cells were incubated with 200μl of a serially 2-fold diluted preparation of 1000 I.U./ml of human alpha or mouse interferon respectively for 1 hour. The interferon-treated cells were then challenged with Vesicular Stomatitis virus at an M.O.I. of 1. The inhibition of the interferon-induced antiviral state by these compounds was estimated as % inhibition in comparison to the control cells. The results are shown below in Table 1.

TABLE 1

| Compound | % inhibition in human fibroblasts | % inhibition in mouse L cells | % inhibition in mouse A9 cells |
|---|---|---|---|
| Indomethacin (50 μM) | not measurable | not measurable | not measurable |
| Indomethacin (300 μM) | not measurable | N.D. | N.D. |
| Oxyphenylbutazone (50 μM) | not measurable | not measurable | 25%* |
| N.D. = Not done | | | |

*This degree of inhibition is too low to be scored as an inhibition because the antiviral assay of interferon is not sensitive enough to differentiate differences of up to 25%.

Reference Example 2: The rapidity of the induction of the antiviral state in human fibroblasts by human α interferon

Human fibroblasts grown to confluency in 96 well Micro-test II dishes were incubated with 200 μl of 100 I.U./ml of human alpha interferon in growth medium containing 10% fetal calf serum at 37° C. Thereafter the cells were challenged with Vesicular Stomatitis virus at an M.O.I. of 1 to test if the treatment conferred an antiviral state on the fibroblast. The results are shown below in Table 2. (-) score indicates absence of an antiviral state, (+) score indicates presence of an antiviral state, (±) score indicates partial resistance to the viral challenge.

TABLE 2

| Time of exposure to interferon | interferon-induced Antiviral state |
|---|---|
| 0 min | - |
| 15 min | ± |
| 30 min | + |
| 60 min | + |

Example 1: Suppression of the action of Type I interferon (α and β) and Type II interferon (γ)

Primary human cell cultures were derived from explants of human foreskins and maintained in vitro as human tissue culture cells. Since the interferon-induced antiviral state is a characteristic cellular response of interferon treatment, it was used as an assay in this search. The assay follows the method of Armstrong as adapted for human cells by Tan.

Human foreskin fibroblast were grown to confluency in 96 well Micro-test II plates as previously described (Tan). The cells were maintained in a growth medium composed of Minimal Eagle's medium and the requisite amount of antibiotic, glutamine and sodium bicarbonate, in a $CO_2$ incubator. The cells were exposed to growth medium containing 1% or 10% fetal calf serum and 50 μM of arachidonic acid, 5,8,11,14-eicosatetraynoic acid, 5,8,11-eicosatriynoic acid, ketoconazole, nordihydroguaiaretic acid and quercetin for 6 hours. The compounds were omitted in the controls.

The cells were then used to assay for the antiviral activity of a given preparation of interferon. The washed cells were incubated with 200 μl of a serially diluted (2-fold) preparation of a 1000 I.U./ml of human alpha interferon for 1 hour at 370 °C. Interferon was then removed and the cells challenged with Vesicular Stomatitis at a M.O.I of 1. The inhibition of the interferon-induced antiviral state was estimated as % inhibition compared to the corresponding control cells. The results are shown below in Table 3.

The results show that arachidonic acid, 5,8,11,14-eicosatetraynoic acid and 5,8,11-eicosatriynoic acid as well as ketoconazole, nordihydroguaiaretic acid and quercetin inhibited the action of interferon by 90% or more. Arachidonic acid, 5,8,11,14-eicosatetraynoic acid and ketoconazole did not inhibit total cellular protein synthesis although they partially inhibited the uptake of $^3$[H]-uridine by the cells, implying cell membrane changes.

TABLE 3

| Compound | % inhibition of antiviral state induced by: | | |
|---|---|---|---|
| | human α interferon | human β interferon | human γ interferon |
| arachidonic acid* (50μM) | ≥90 | ≥90 | ≥80 |
| 5,8,11,14-eicosatetraynoic acid* (50μM) | ≥90 | ≥90 | ≥80 |
| 5,8,11,-eicosatriynoic acid* (50μM) | ≥90 | N.D. | N.D. |
| Nordihydroguaiaretic acid ** (50μM) | ≥90 | ≥90 | N.D. |
| Ketoconazole** (100μM) | ≥90 | ≥90 | N.D. |
| Quercetin** (50μM) | 90 | N.D. | N.D. |
| N.D. = Not done | | | |

* = 1% fetal calf serum
** = 10% fetal calf serum

The procedure was repeated to investigate the effect on the inhibitory action of arachidonic acid and 5,8,11,14-eicosatetraynoic acid of increasing the concentration of serum from 1% to 10% and of increasing

the concentrations of inhibitor. The results are shown below in Table 4. Increasing the serum concentration reversed the inhibitory effect of arachidonic acid and its analogue but could be counteracted by further increasing the concentration of the arachidonic acid or 5,8,11,14-eicosatetraynoic acid. At the concentrations tested, and the accompanying concentration of serum, no gross cytotoxicity was observed with arachidonate and its analogues.

TABLE 4

| Compound | Concentration of fetal calf serum in growth medium containing compound | % inhibition of the antiviral state induced by human α interferon |
|---|---|---|
| arachidonic acid (50 µM) | 1% | ≧90 |
| arachidonic acid (50 µM) | 10% | 0 |
| arachidonic acid (300 µM) | 10% | ≧90 |
| 5,8,11,14-eicosatetraynoic acid (50 µM) | 1% | ≧90 |
| 5,8,11,14-eicosatetraynoic acid (50 µM) | 10% | 0 |
| 5,8,11,14-eicosatetraynoic acid (200 µM) | 10% | ≧90 |

Example 2: Kinetics of the inhibition of the interferon-induced antiviral state by arachidonic acid in human fibroblasts

Human fibroblasts grown in 96 well Micro-test II plates were incubated with growth medium containing 1% fetal calf serum and 50µM arachidonic acid for up to 6 hours. The cells were then assayed for the inhibition of the human alpha interferon-induced antiviral state. The results are shown in Figure 1. These show that arachidonic acid inhibits the interferon-induced antiviral state within minutes.

Also, human fibroblasts in 96 well Micro-test II plates were incubated with growth medium containing 1% fetal calf serum and 50µM arachidonic acid for 6 hours. The cells were washed twice with 200µ1 of growth medium containing 1% fetal calf serum to remove most of the arachidonic acid and reincubated with growth medium containing 1% fetal calf serum for 0.5, 1, 2 or 24 hours at 37°C before they were used in the study for the inhibition of human alpha interferon-induced antiviral state. The results are shown in Figure 2. These show that the inhibition by arachidonic is reversible with time.

Example 3: Elucidating the mechanism of action of the suppressive effect

The binding of interferons to human cells was analysed with human alpha interferon labelled with radioactive iodine. The $^{125}$I-labelled human alpha interferon was assayed for biological activity as well as analysed by sodium dodecyl sulphate (SDS)-polyacrylamide gel electrophoresis before use to ensure that the labelled ligand was not degraded and biologically active. The labelled ligand was then used in binding studies with human fibroblasts as previously described (Thompson et al., Branca and Baglioni; and Epstein et al.).

The results of this study are expressed as a Scatchard plot of the binding data in Figures 3 to 6. The cells were washed twice with growth medium to remove most of the fatty acid before they were used to measure the binding of $^{125}$I-human alpha interferon to their receptors. B and F are concentration of bound and free $^{125}$I-human alpha interferon respectively. Human cells were exposed to growth medium with:

- Figure 3: 1% fetal calf serum containing 50µM of arachidonic acid (▲), 50 µM 5,8,11,14-eicosatetraynoic acid (□) or growth medium with 1% fetal calf serum (■);
- Figure 4: 10% fetal calf serum containing 300 µM sodium arachidonate (■) or growth medium with 10% fetal calf serum (□);
- Figure 5: 10% fetal calf serum containing 200 µM 5,8,11,14-eicosatetraynoic acid (■) or growth medium with 10% fetal calf serum (□), and
- Figure 6: 10% fetal calf serum containing 10µM (□) or 50uM (■) nordihydroguaiaretic acid and growth medium with 10% fetal calf serum (▲).

The presence of arachidonic acid or 5,8,11,14-eicosatetraynoic acid in growth medium containing 1% fetal calf serum altered the binding constant of interferon to its receptor (Figures 3 and 4) as well as down regulating the interferon receptors by about 30-60% (Figures 4 and 5). The $K_d$ of interferon binding to human fibroblasts estimated as $1.59 \times 10^{-10}$M was increased to $3.70 \times 10^{-10}$M and $3.25 \times 10^{-10}$M in cells treated with 33µM arachidonic acid or 5,8,11,14-eicosatetraynoic acid, respectively in the presence of 1% fetal calf serum (Figure 3).

Where the cells were treated with sodium arachidonate or 5,8,11,14-eicosatetraynoic acid in growth medium containing 10% fetal calf serum, the concentrations of arachidonate and 5,8,11,14-eicosatetraynoic acid had to be increased to 300µM and to 200µM respectively to overcome the serum effect before they could inhibit the interferon-induced antiviral state by ≥90%. The $K_d$ obtained are $2.40 \times 10^{-10}$M and $3.17 \times 10^{-10}$M respectively compared to a $K_d$ of $1.99 \times 10^{-10}$M and $1.44 \times 10^{-10}$M for their concurrent cell control (Figures 4 and 5). The $K_d$ for the nordihydroguaiaretic acid-treated cells is also estimated to increase from $2.17 \times 10^{-10}$M to $3.38 \times 10^{-10}$M (Figure 6).

Increases of $K_d$ values for the treated cells imply a diminished binding of ligands (interferon) to their receptors to the inhibitor-treated cells. Nordihydroguaiaretic acid does not seem to significantly down regulate the receptors. These data suggest that arachidonic acid, 5,8,11,14-eicosatraynoic acid, sodium arachidonate and nordihydroghuaiaretic acid can diminish the binding of ligands (interferon) to their receptors, inhibiting the interferon-induced effects on cells.

Example 4: Effect of arachidonic acid and 5,8,11,14-eicosatetraynoic acid (ETYA) on interferon-induced transcription of 6/16 gene

When cells are treated with interferon several genes are transcribed. One of them is the 6/16 gene. We studied the effects of arachidonic acid and 5,8,11,14-eicosatetraynoic acid on the transcription of the 6/16 gene in cells stimulated by human interferon. Normal human fibroblasts were incubated for 6 hours with MEM medium containing 1% fetal calf serum (lanes 1 and 2) and in addition 50μM ETYA (lanes 3 and 4) or 30μM arachidonic acid (lanes 5 and 6). Following this, the cells were then incubated for 2 hours with growth medium containing 1% fetal calf serum (lanes 1,3 and 5) or 10 I.U./ml of human alpha interferon (lanes 2, 4 and 6). A total 20μg of RNA obtained from each treatment was electrophoresed on formaldehyde-agarose gels and the Northern blots hybridized to $P^{32}$ labelled 6/16 cDNA.

The results are shown in Figure 7. As expected, the transcription of this gene is strongly inhibited. This result is consistent with the results demonstrating that arachidonates down regulate interferon receptors as well as affecting the binding of interferon to its receptor, resulting in non-expression of the interferon-induced genes.

References

Abdi, E.A., Kamitomo, V.J., McPherson, T.A., Konrad, M.W., Inoue, M. and Tan, Y.N. (1986) Clin. Invest. Med. 9, 33-40.

Bloom, M. (1987) Science 235, 154-155.

Branca, A.A. and Baglioni, C. (1981) Nature 294, 768-770.

Dore-Duffy, P., Perry, W. and Kuo, H-H. (1983) Cell. Immunol. (1979) 232-239.

Douglas, R.M., Moore, B.W., Miles, H.B., Davies, L.M., Graham, N.M.H., Ryan, P., Worswick, D.A. and Albrecht, J.K. (1986) New Eng. a. Med. 314, 65-70.

Duff, G. (1985) Nature 313, 352-353.

Eldor, A., Fridman, R., Vlodavsk, I., HY-AM, E. Fuks, Z. and Panet A. (1984) J. Clin. Invest. 73, 251-257.

Elliot, K.R., Princler, G.L., Urba, W.J. And Faltynek, C.R. (1988) J. Cell Physiol. 134, 85-92.

Epstein, C.J., McManus, N.H., Epstein, L.B., Branca, A.A., D'Alessandro, S.B and Baglioni, C. (1982) Biochem. Biophys. Res. Comm. 107, 1060-1066.

Faltynek, C.R., Princler, G.L., Gusella, G.L., Varesio, L., and Radzioch, D. (1989) J. Biol. Chem. 264, 13405-4311.

Fuse, A, Mahmud, I. and Kuwata, T. (1978) Cancer Res. 42, 3209-3214.

Hayden, F.G., Mills, S.E. and Johns, E.M. (1983) J. Infect. Dis. 148, 914-921 (a).

Hayden, F.G., Albrecht, J.K., Kaiser, D.L., and Gwaltney, J.M. (1986) New Eng. J. Med. 314, 71-75 (b).

Kantor, T.G. (1987) Am. J. Med. 83, 2-5.

McCoy, E.E., and Strynadka, K. (1987) Prog. Clin. Biol. Res. 246, 221-230.

McPherson, T.A. and Tan, Y.H. (1980) J. Natl. Cancer Inst. 65, 75-79.

Neilsen, O.H., Elmgreen, J. and Ahnfelt-Ronne, I. (1988) Inflammation 12, 169-179.

Pfeffer, L.M., and Reich, N. (1989) J.Ifn. Res. 9, supplement 2, S156.

Pottathil, R., Chandrabose, K.A., Cuatrecasas, P., and Lang, D.J. (1980). Proc. Natl. Acad. Sci. USA 177, 5437-5440.

Sarkar, F.H., and Gupta, S.L. (1982). Virology 123, 448-451.

Scott, G.M, Secher, D.S., Flowers, D., Bate, I., Cantell, K. and Tyrell, D.A.J. (1981) Br. Med. J. 282, 1345-1348.

Takaoki, M., Yamashita, Y., Koike, K. and Matsuda, S. (1988). J. Ifn. Res, 8, 727-733.

Tan, Y.H. (1975) Nature 253, 280-282.

Tan, Y.H. Yap, W.H., Lim, A., Sim, T., and Menon, S.D. (1990) J. Cell Biochem. 14B:266

Thompson, M.R., Zhang, Z., Fournier, A. and Tan, Y.H. (1985) J. Biol. Chem. 260, 563-367.

Yap, W.H., Teo, T.S., and Tan, Y.H. (1986) Science 234, 355-358.

Yap, W.H., Teo, T.S., McCoy, E., and Tan, Y.H. (1986) Proc. Natl. Acad. Sci. USA 83, 7765-7769.

Yaron, M., Yaron, I., Gurari-Rotman, D., Revel, M., Linder, H.R. and Zor, V. (1977) Nature 267, 457-459.

## Claims

1. Use, in the preparation of a medicament for use in the treatment of a disease state associated with

the endogenous presence and/or production of a cytokine, of arachidonic acid or an analogue thereof capable of interfering with the binding of the cytokine to the cellular receptor for the cytokine.

2. The use according to claim 1, wherein the cytokine is type I or II interferon.

3. The use according to claim 2, whereing the analogue is capable of producing a percentage inhibition of the interferon-induced antiviral state of at least 50%.

4. The use according to any one of the preceding claims, wherein a $C_{18}$-$C_{22}$ monocarboxylic polyunsaturated fatty acid or a pharmaceutically acceptable salt, $C_1$-$C_4$ alkyl ester, amide or mono-($C_1$-$C_4$ alkyl) amide thereof is used in the preparation of the medicament.

5. The use according to any one of claims 1 to 3, wherein a compound selected from 5,8,11,14-eicosatetraenoic acid, 5,8,11,14-eicosatetraynoic acid, 5,8,11-eicosatriynoic acid, linoleic acid, cis-1-acetyl-4-{4-[2-(2,4-dichlorophenyl)-2-imidazol-1-ylmethyl-1,3-dioxolan-4-ylmethoxy]phenyl}-piperazine; 4,4′-(2,3-dimethyltetramethylene)bis(benzene-1,2-diol); and

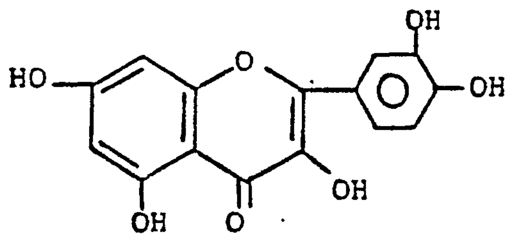 ;

and, where appropriate, pharmaceutically acceptable salts thereof is used in the preparation of the medicament.

6. Use, in the preparation of a medicament for use in alleviating the symptoms of the common cold or of influenza, of arachidonic acid or an analogue thereof capable of producing a percentage inhibition of the interferon-induced antiviral state of at least 50%.

7. The use according to claim 6, wherein a $C_{18}$-$C_{22}$ monocarboxylic polyunsaturated fatty acid or a pharmaceutically acceptable salt, $C_1$-$C_4$ alkyl ester, amide or mono-($C_1$-$C_4$ alkyl) amide thereof is used in the preparation of the medicament.

8. The use according to claim 6, wherein a compound selected from 5,8,11,14-eicosatetraenoic acid, 5,8,11,14-eicosatetraynoic acid, 5,8,11-eicosatriynoic acid, linoleic acid, cis-1-acetyl-4-{4-[2-(2,4-dichlorophenyl)-2-imidazol-1-ylmethyl-1,3-dioxolan-4-ylmethoxy]phenyl}-piperazine; 4,4′-(2,3-dimethyltetramethylene)bis(benzene-1,2-diol); and

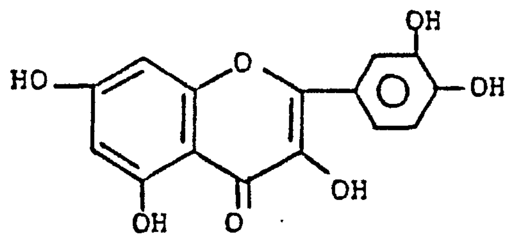 ;

and, where appropriate, pharmaceutically acceptable salts thereof is used in the preparation of the medicament.

FIGURE 1

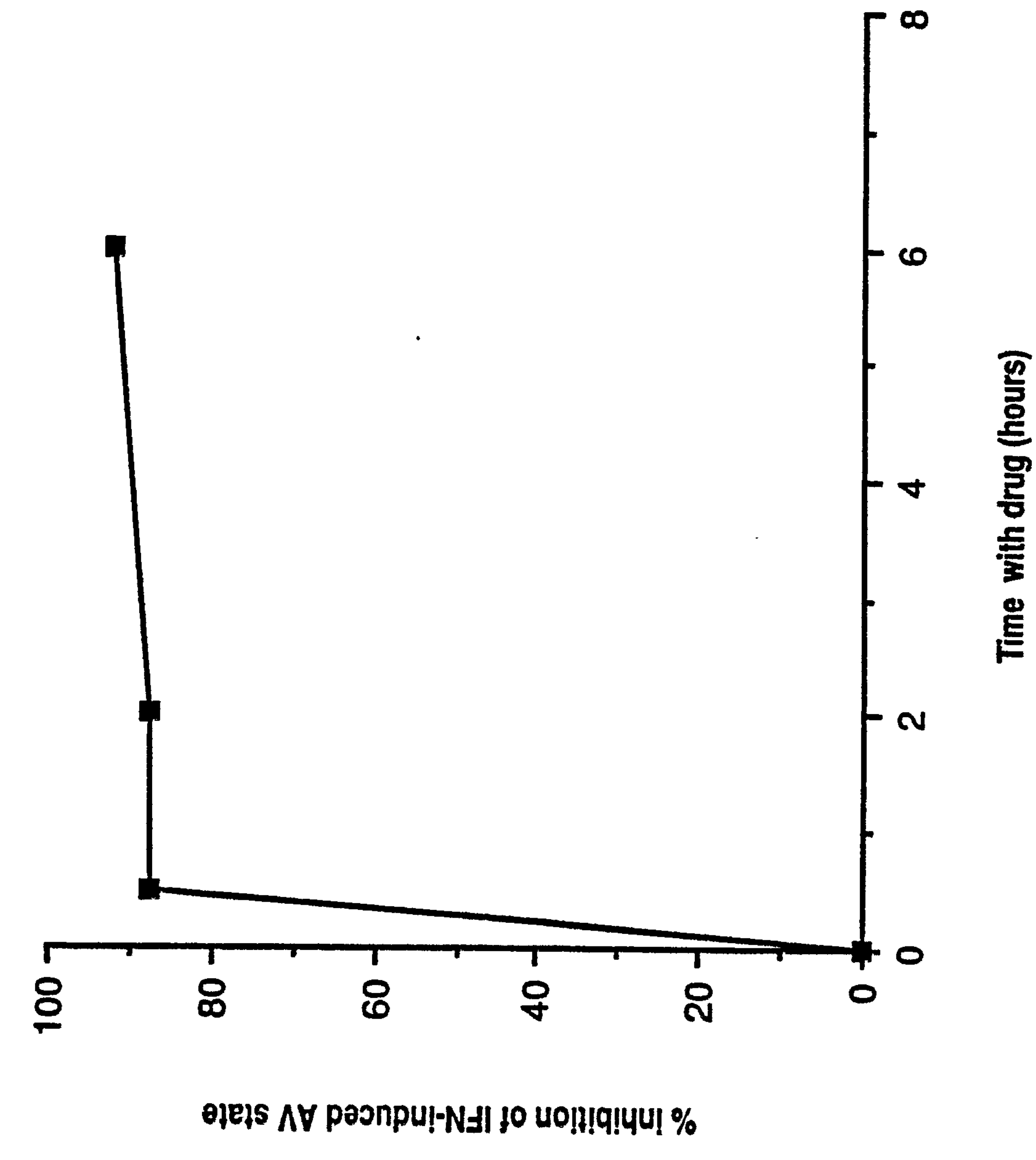
% inhibition of IFN-induced AV state
100
80
60
40
20
0
Time with drug (hours)
0
2
4
6
8

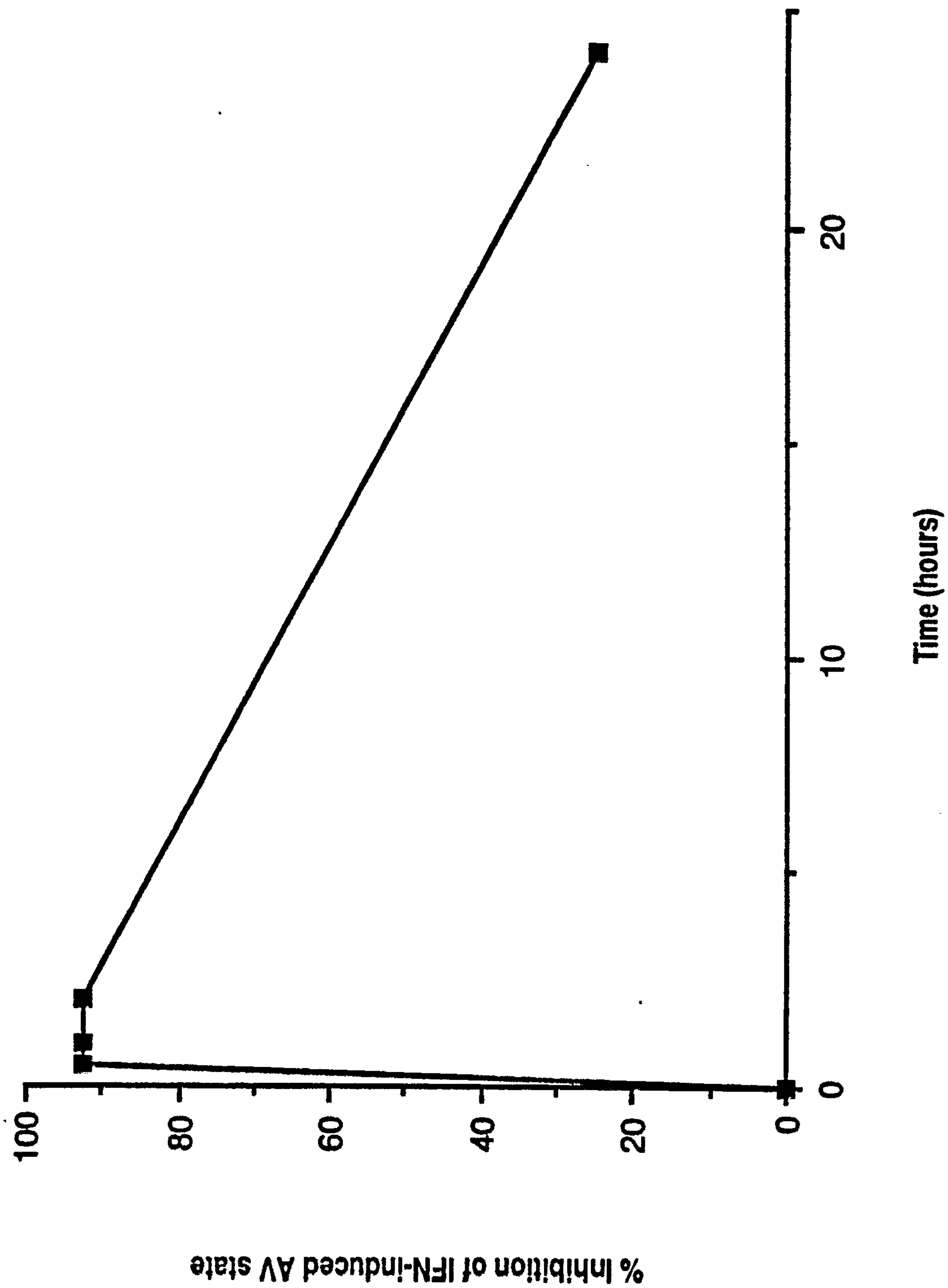
% Inhibition of IFN-induced AV state
100
80
60
40
20
0
0
10
20
Time (hours)

Figure 2

FIGURE 3

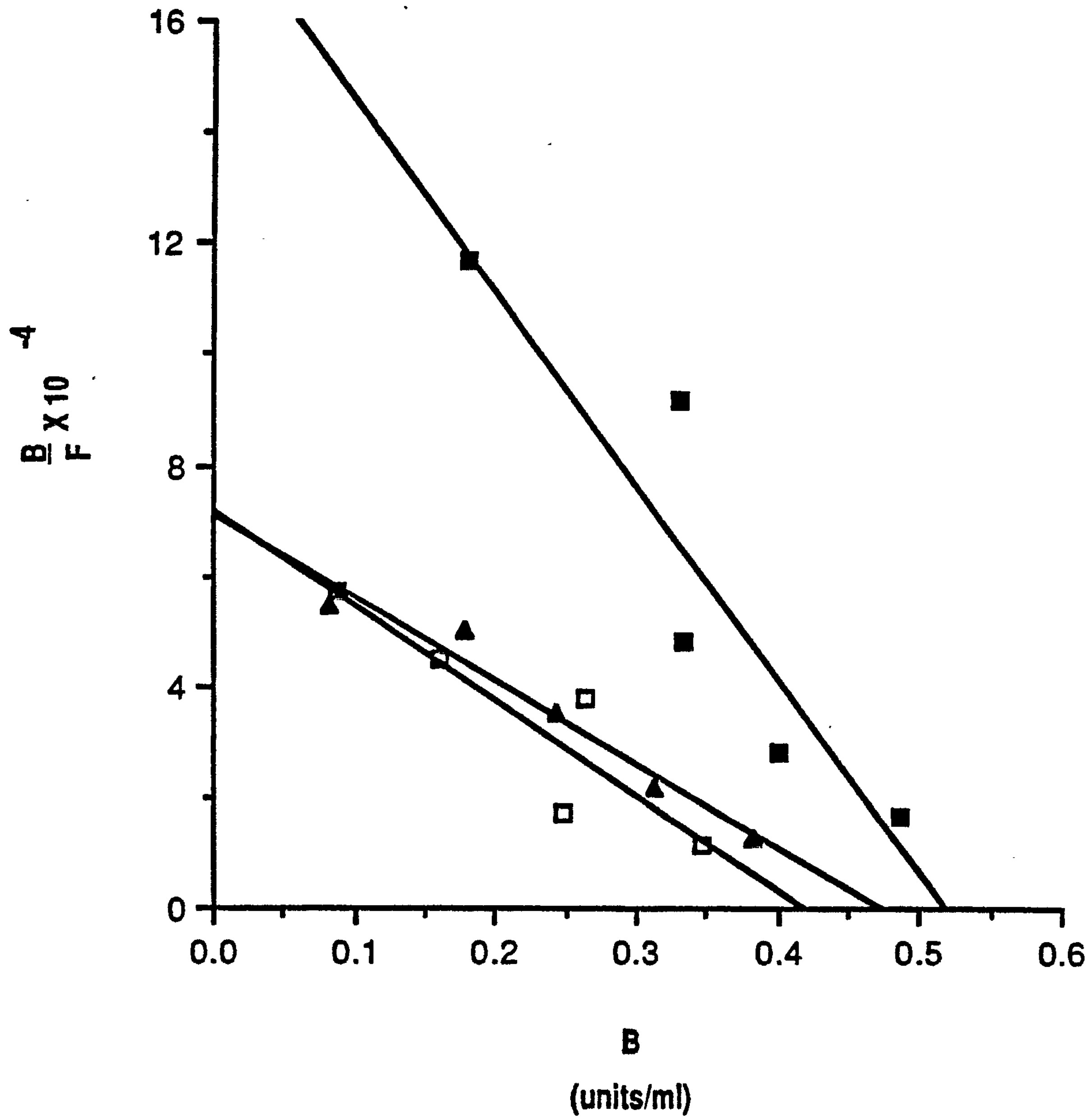

FIGURE 4

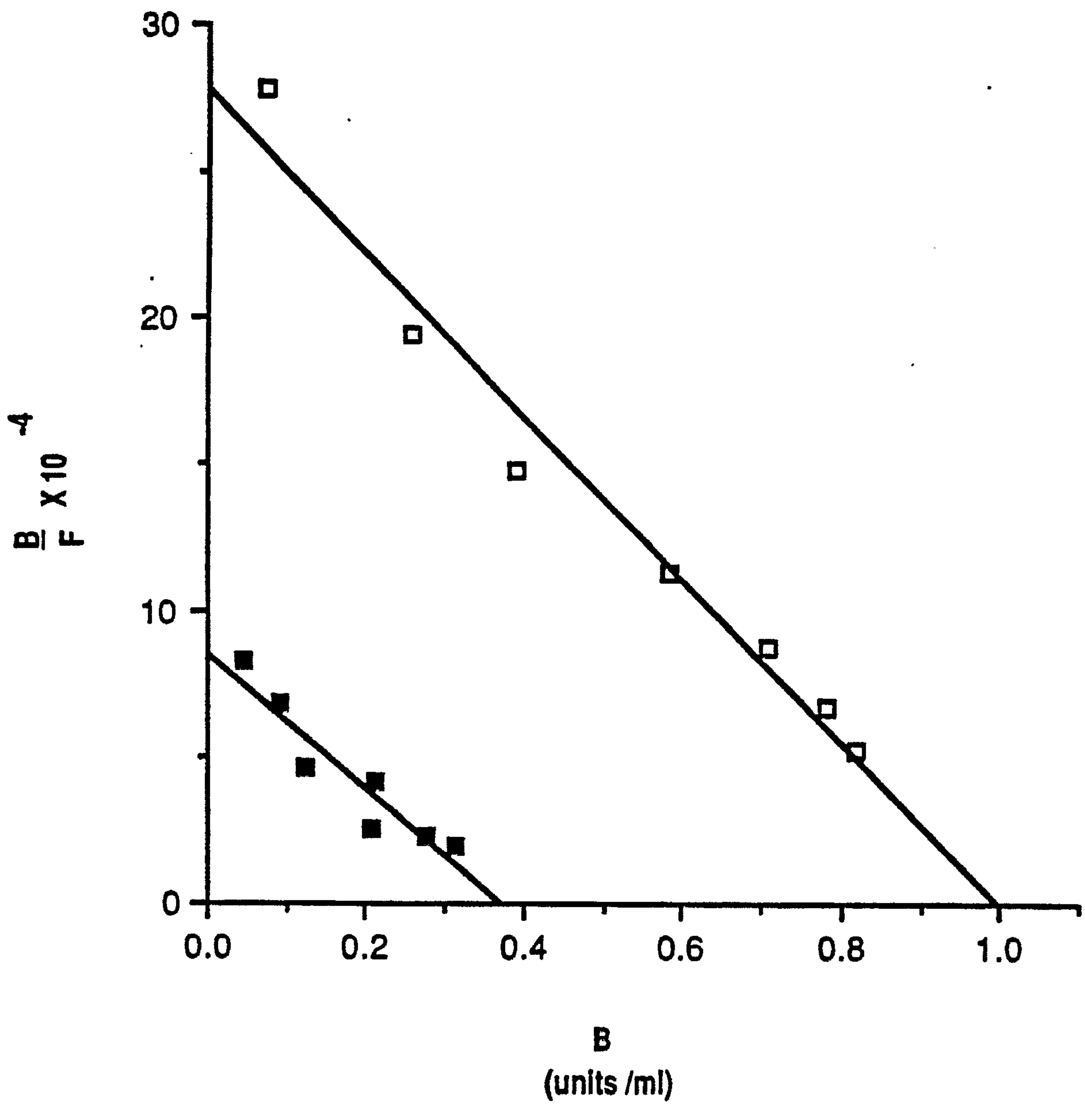

FIGURE 5

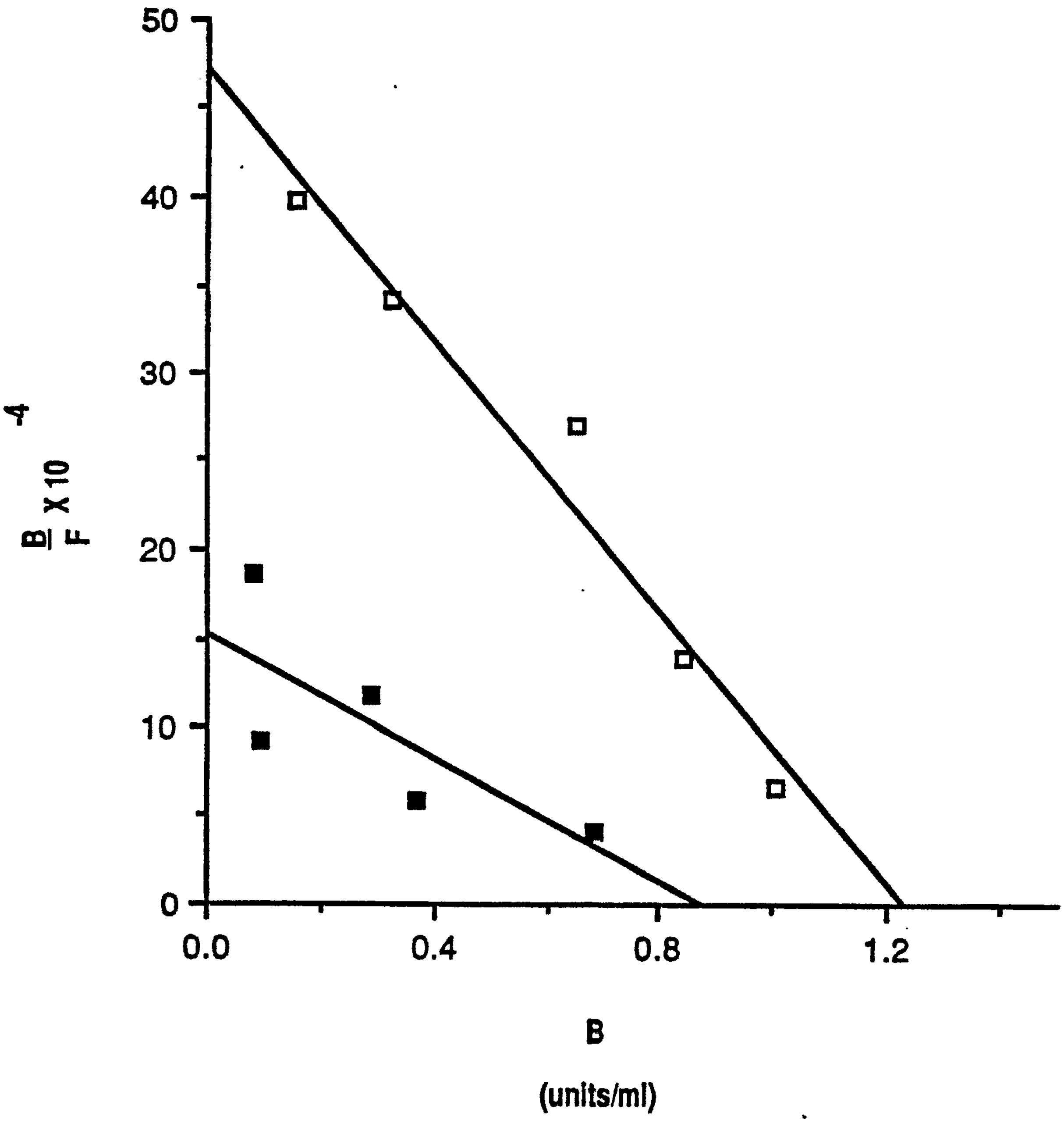

FIGURE 6

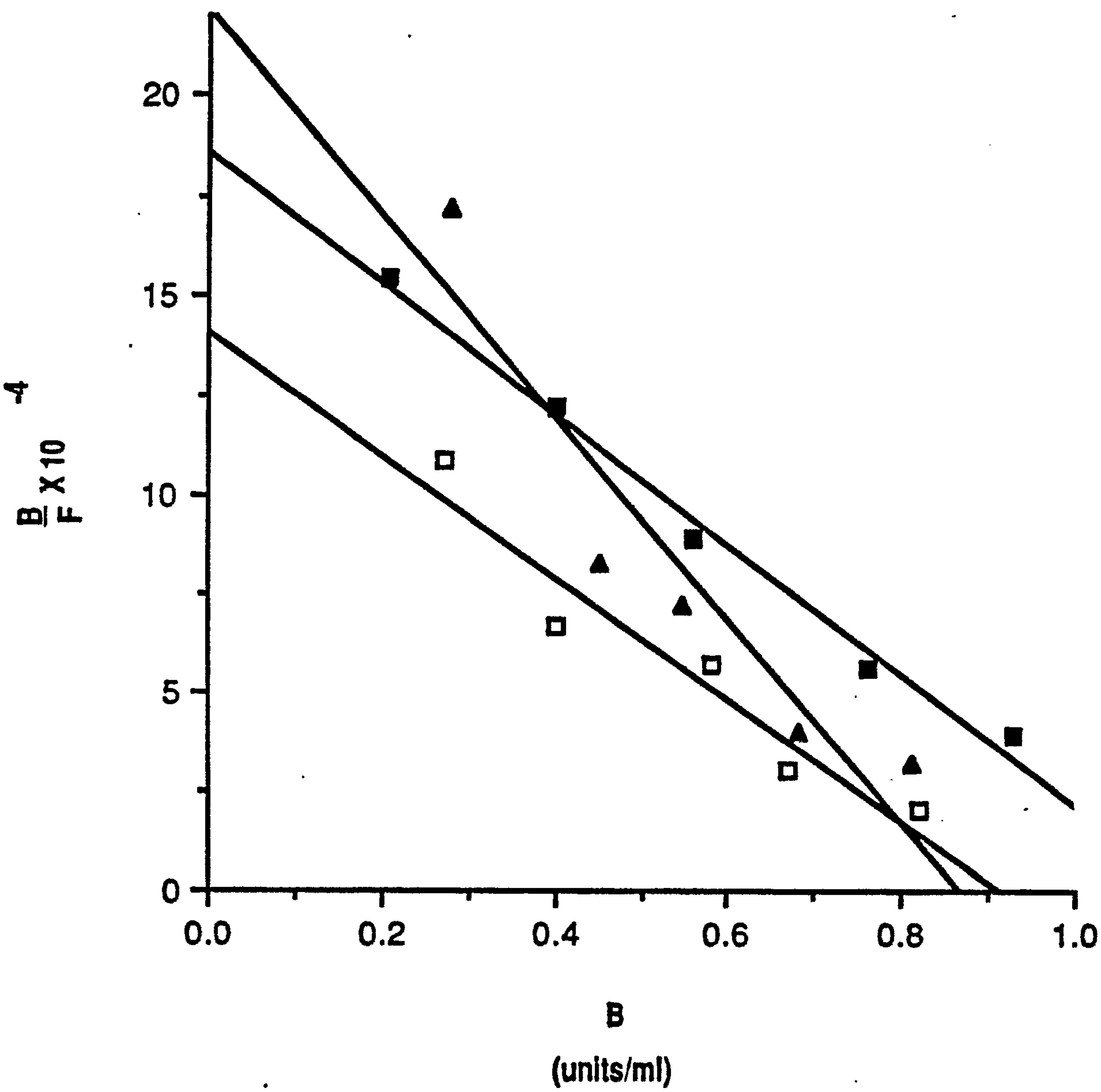

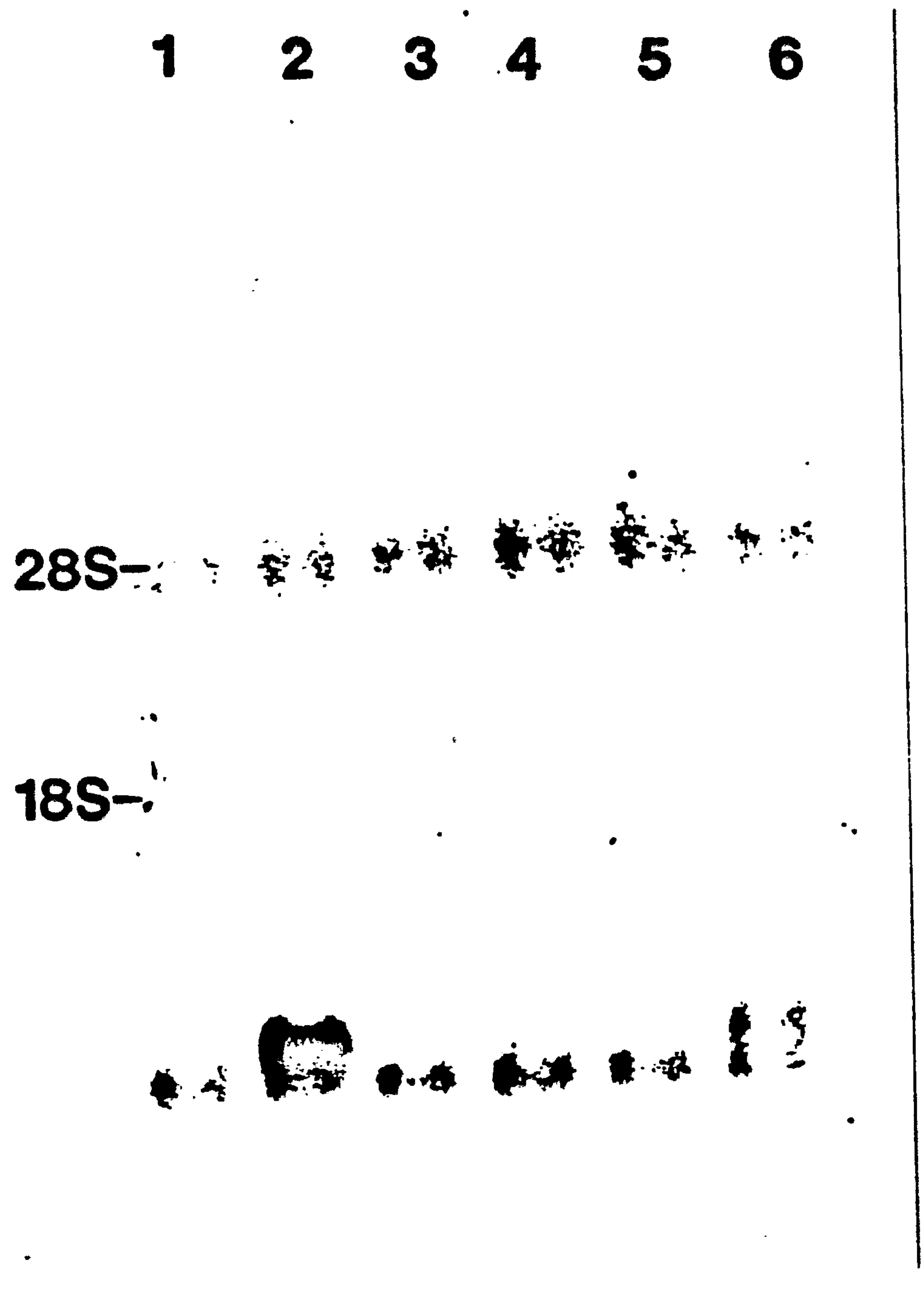


FIGURE 7